(19) **Europäisches Patentamt / European Patent Office / Office européen des brevets**

(11) **EP 4 427 696 B1**

(12) **EUROPEAN PATENT SPECIFICATION**

(45) Date of publication and mention
of the grant of the patent:
**30.04.2025 Bulletin 2025/18**

(21) Application number: **23218386.3**

(22) Date of filing: **20.12.2023**

(51) International Patent Classification (IPC):
***A61B 34/10*** *(2016.01)*

(52) Cooperative Patent Classification (CPC):
**A61B 34/10; A61B 6/505; G06T 7/60; G06T 7/73;**
A61B 2034/104; G06T 2207/10116;
G06T 2207/20084; G06T 2207/30008

(54) **AUTOMATIC PLANNING METHOD AND SYSTEM FOR SIMULATED KNEE SURGERY BASED ON DEEP LEARNING**

AUTOMATISCHES PLANUNGSVERFAHREN UND SYSTEM FÜR SIMULIERTE KNIECHIRURGIE AUF DER BASIS VON TIEFENLERNEN

PROCÉDÉ ET SYSTÈME DE PLANIFICATION AUTOMATIQUE POUR UNE CHIRURGIE SIMULÉE DU GENOU SUR LA BASE D'UN APPRENTISSAGE PROFOND

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **09.03.2023 CN 202310221343**

(43) Date of publication of application:
**11.09.2024 Bulletin 2024/37**

(73) Proprietor: **Tianjin Hospital**
**Tianjin (CN)**

(72) Inventors:
• **MA, Xinlong**
**Tianjin (CN)**
• **MA, Jianxiong**
**Tianjin (CN)**
• **WU, Zhichao**
**Tianjin (CN)**

• **BAI, Haohao**
**Tianjin (CN)**
• **WANG, Ying**
**Tianjin (CN)**
• **LU, Bin**
**Tianjin (CN)**
• **SUN, Lei**
**Tianjin (CN)**
• **JIN, Hongzhen**
**Tianjin (CN)**
• **LI, Yan**
**Tianjin (CN)**

(74) Representative: **Sun, Yiming**
**HUASUN Patent- und Rechtsanwälte**
**Friedrichstraße 33**
**80801 München (DE)**

(56) References cited:
**WO-A1-2022/007342    CN-A- 113 919 020**
**CN-A- 114 711 796    US-A1- 2023 058 837**

**Description**

**TECHNICAL FIELD**

The present disclosure belongs to the technical field of data processing, and particularly relates to an automatic planning method and system for a simulated knee surgery based on deep learning.

**BACKGROUND**

Knee osteoarthritis is one of main upsetting orthopedic diseases to people. In its clinical surgeries, a high tibial osteotomy (HTO) and a distal femoral osteotomy (DFO) are used to adjust an abnormal mechanical axis of a lower limb, thus prolonging a service life of the knee. Preoperative diagnosis and preoperative planning depend on a lower limb full-length X-ray image of the patient. It is time-consuming for a professional doctor to determine key points in the knee surgery. The workload for planning a surgery scheme limits a number of patients to be treated by the doctor in unit time.

Document WO 2022/007342 A1 discloses an image processing method which includes the steps of: Obtain the target key point of the target image; segment the target object in the target image to obtain the segmentation result of the target object; obtain the processing type of the target object; according to the target key point, the segmentation result and the processing type determines at least one processing parameter of the target object.

**SUMMARY OF PRESENT INVENTION**

The invention is defined by the appended independent claims.

Further, the selecting a key regional image in the medical image, and performing object detection on the key regional image to extract key points comprises:

selecting an image including a hip region, a knee region and an ankle region as the key regional image;
performing, with a you only look once X (YOLOX), the object detection on the image including the hip region, the knee region and the ankle region;
extracting a hip center, a hinge point in the knee region, a Fujisawa point, a surgery point and an ankle center as the key points with a pulse feature disentanglement network (PFDNet).

Further, the calculating a corrective angle with coordinates of the extracted key points by labeling the key points and a mechanical axis comprises:

assuming that a connecting line from the hip center to the Fujisawa point and an extended line form a target mechanical axis called a line 1, and the hinge point and the ankle center form a line 2, then making rotation to the line 1 with the hinge point as a center and a length of the line 2 as a radius to obtain a line 3, where the ankle center coincides with the line 1, and an included angle between the line 3 and the line 2 is an angle to be corrected in the HTO; and assuming that a connecting line from the ankle center to the Fujisawa point and an extended line form a target mechanical axis called a line 1', and the hinge point and the hip center form a line 2', then making rotation to the line 1' with the hinge point as a center and a length of the line 2' as a radius to obtain a line 3', where the hip center coincides with the line 1', and an included angle between the line 3' and the line 2' is an angle to be corrected in the DFO.

The automatic planning method and system for a simulated knee surgery based on deep learning provided by the present disclosure has the following advantages over the prior art: The present disclosure provides the planning method and system for a surgery around the knee with coordinate calculation and image processing, and automatically processes the lower limb full-length X-ray image with a regional object detection algorithm and a key point detection algorithm, thereby realizing simulation on eight surgeries around the knee. The present disclosure can effectively shorten manual point selection time by automatically extracting the key points, and can effectively shorten surgery planning time by automatically simulating surgery planning and displaying an effect. Therefore, the present disclosure significantly improves diagnosis time of each patient, greatly reduces the reliance on the professional doctor, alleviates a burden of the doctor, and achieves a higher diagnosis and treatment efficiency.

**BRIEF DESCRIPTION OF THE DRAWINGS**

As a part of the present disclosure, the accompanying drawings of the specification provide further understanding of the present disclosure. The schematic embodiments of the present disclosure and description thereof are intended to explain the present disclosure and are not intended to constitute an improper limitation to the present disclosure. In the drawings:

FIG. 1 schematically shows an automatic planning method for a simulated knee surgery based on deep learning according to the present disclosure;

FIG. 2 schematically shows a ruler image according to the present disclosure;

FIG. 3 schematically shows a detection result of an LSD according to the present disclosure;

FIG. 4 schematically shows an original mechanical axis of a lower limb according to the present disclosure;

FIG. 5 schematically shows a predicted result according to the present disclosure;

FIG. 6 shows a hip region according to the present disclosure;

FIG. 7 shows a knee region according to the present disclosure;

FIG. 8 shows an ankle region according to the present disclosure;

FIG. 9 shows planning of a medial opening HTO according to the present disclosure;

FIG. 10 shows a method for calculating an included angle between straight lines according to the present disclosure;

FIG. 11 schematically shows rotation of an image from a rectangular coordinate system to a polar coordinate system according to the present disclosure;

FIG. 12 shows an effect of a medial opening HTO according to the present disclosure;

FIG. 13 shows planning of a lateral opening DFO according to the present disclosure;

FIG. 14 shows an effect of a lateral opening DFO according to the present disclosure; and

FIG. 15 shows various preoperative planning effects of an HTO and a DFO according to the present disclosure.

## DETAILED DESCRIPTION OF THE EMBODIMENTS

It should be noted that the embodiments in the present disclosure and features in the embodiments may be combined in a non-conflicting manner.

It should be understood that in the description of the present disclosure, terms such as "central", "longitudinal", "transverse" "upper", "lower", "front", "rear", "left", "right" "vertical", "horizontal", "top", "bottom", "inside" and "outside" indicate the orientation or positional relationships based on the drawings. They are merely intended to facilitate and simplify the description of the present disclosure, rather than to indicate or imply that the mentioned device or components must have a specific orientation or must be constructed and operated in a specific orientation. Therefore, these terms should not be construed as a limitation to the present disclosure. Moreover, the terms such as "first" and "second" are used only for the purpose of description and cannot be understood as indicating or implying relative importance or implicitly indicating the number of technical features denoted. Thus, features defined with "first" and "second" may explicitly or implicitly include one or more of the features. In the description of the present disclosure, unless otherwise specified, "a plurality of" means at least two.

In the description of the present disclosure, it should be noted that, unless otherwise clearly specified, meanings of terms "install", "connected with", and "connected to" should be understood in a board sense. For example, the connection may be a fixed connection, a removable connection, or an integral connection; may be a mechanical connection or an electrical connection; may be a direct connection or an indirect connection by using an intermediate medium; or may be intercommunication between two components. A person of ordinary skill in the art may understand specific meanings of the above terms in the present disclosure based on a specific situation.

The present disclosure will be described in detail below with reference to the accompanying drawings and the embodiments.

Explanations on technical terms

DFO: distal femoral osteotomy

HTO: high tibial osteotomy

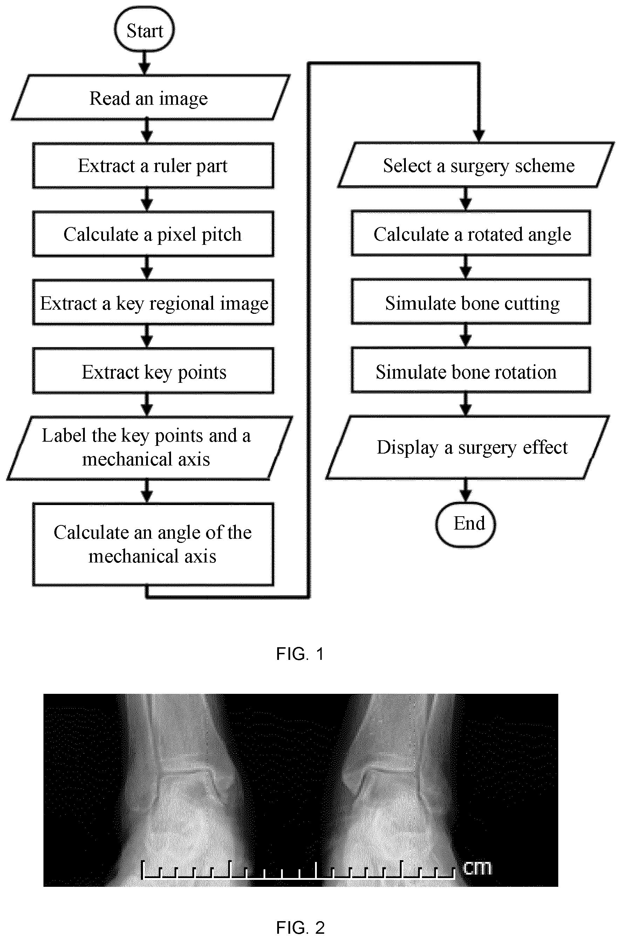

As shown in FIG. 1, the present disclosure provides an automatic planning method for a simulated knee surgery based on deep learning, including the following steps.

In step 1, a medical image is acquired, and the pixel pitch in the medical image is converted into an actual length.

An actual size serves as a criterion in actual surgery. Hence, the pixel pitch in the image is converted into the actual length, namely a pixel pitch is obtained. FIG. 2 shows a ruler image. There are eighteen 10-mm spacings on each ruler.

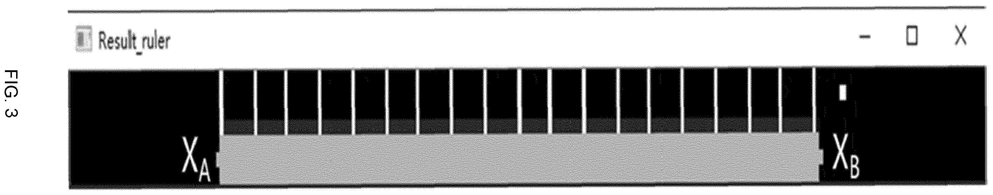

According to characteristics of the image, a pixel threshold is preset, white pixel points in the image are screened out, and the ruler image is detected with an LSD, as shown in FIG. 3. A head endpoint coordinate and a tail endpoint coordinate of the ruler are acquired. Conversion is performed on a pixel difference value between the head endpoint coordinate and the tail endpoint coordinate ($X_A$ and $X_B$) of the ruler in an X direction and a millimeter unit to obtain a coefficient of relationship $\alpha$ between the millimeter unit and a coordinate value, as shown in Eq. (1). Upon the conversion, length information, rather than pixel information, is obtained. According to the criterion in surgery planning, a surgery scheme is selected. The pixel information cannot be used as the criterion:

$$\alpha = \frac{(X_A - X_B)/18}{10} \qquad (1).$$

In step 2, a key regional image in the medical image is selected, and object detection is performed on the key regional image to extract key points.

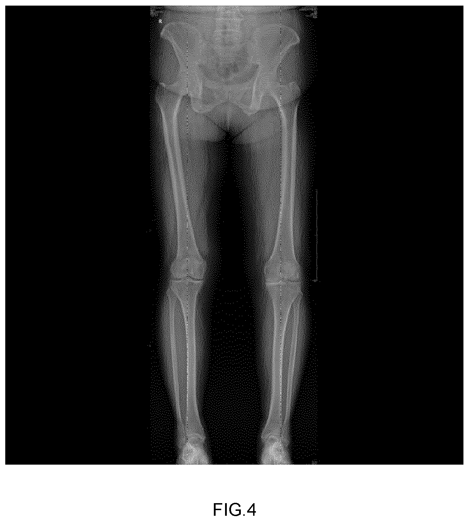

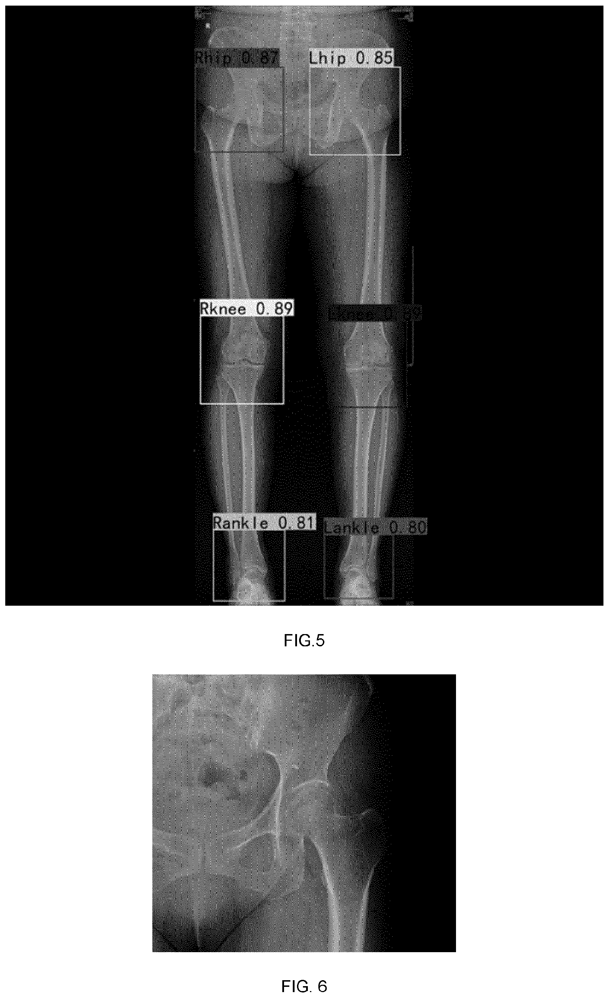

The present disclosure selects a key region including the key points, and does not perform global processing on the whole image. This downsizes the model and reduces the noise. The present disclosure performs the object detection on a hip region, a knee region and an ankle region with a YOLOX in deep learning. As shown in FIGS. 4-5, FIG. 4 shows an original lower limb X-ray image transmitted to the YoloX, and FIG. 5 shows a result upon regional selection of the YoloX network. FIGS. 6-8 show a selected key region, and uses a left lower limb as an example.

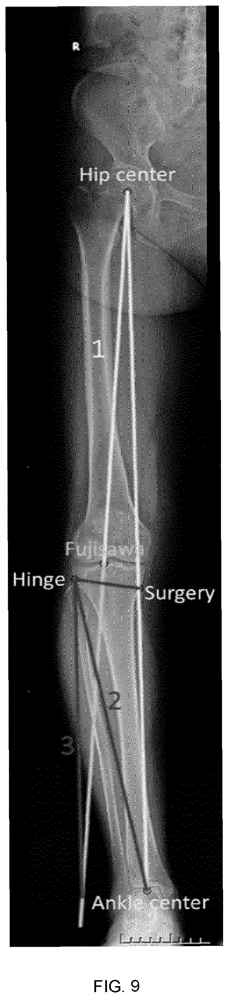

In the key regional image, a hip center, a hinge point in the knee region, a Fujisawa point, a surgery point and an ankle center are extracted with a PFDNet. As shown in FIG. 9, the extracted key points include the hip center, the hinge point, the surgery point and the ankle center.

In step 3, a corrective angle is calculated with coordinates of the extracted key points by labeling the key points and a mechanical axis, a rotated angle is calculated, bone cutting and bone rotation are simulated to obtain a surgery planning image, and automatic planning is performed on an HTO and a DFO.

The HTO and the DFO are planned with a coordinate of the hip center, a coordinate of the ankle center, a coordinate of the hinge point, a coordinate of the surgery point and a coordinate of the Fujisawa point. As shown in FIG. 9, to select a correcting mechanical axis and implement image rotation in four small surgeries in the HTO, based on the surgery point, a lower half part of the image is rotated with the hinge point as a center. The present disclosure takes the medial opening HTO as an example. A connecting line from the hip center to the Fujisawa point and an extended line form a target mechanical axis called a line 1. The hinge point and the ankle center forms a line 2. With the hinge point as a center, and a length of the line 2 as a radius, rotation is made to the line 1 to obtain a line 3. In this case, the ankle center coincides with the line 1 (target mechanical axis). An included angle between the line 3 and the line 2 is an angle to be corrected in the medial opening HTO.

Specifically, assuming that the line 1 (target mechanical axis) determined by the hip center and the Fujisawa center has a linear equation as shown in Eq. (2), where, $(x_0, y_0)$ is a coordinate of the hinge point, $(x_1, y_1)$ is a coordinate of a parameter point for determining the line 1, and $(x_2, y_2)$ is a coordinate of the original ankle center, then:

$$y_1 = k_1 x_1 + b_1 \qquad (2).$$

The length of the line 2 is given by a Euclidean distance Eq. (3):

$$D = \sqrt{(x_0 - x_2)^2 + (y_0 - y_2)^2} \tag{3}.$$

As shown in Eq. (4), assuming that the rotated ankle center has a coordinate $(x_3, y_3)$, a distance from the hinge point $(x_0, y_0)$ to the rotated ankle center is given by:

$$D' = \sqrt{(x_0 - x_3)^2 + (y_0 - y_3)^2} \tag{4}.$$

As shown in Eq. (5), with the linear equation of the target mechanical axis as a constraint condition, the rotated ankle center has a coordinate given by:

$$(x_3, y_3) = \begin{cases} D' = \sqrt{(x_0 - x_3)^2 + (y_0 - y_3)^2} \\ y_1 = k_1 x_1 + b_1 \end{cases} \tag{5}.$$

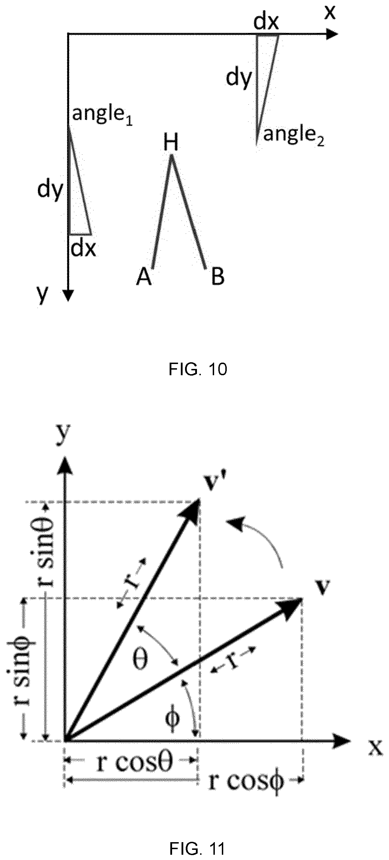

In the present disclosure, the angle is calculated as shown in FIG. 10. $\angle AHB$ represents a to-be-calculated angle at any position in a plane, such as an angle formed by the Fujisawa point, the hip center and the ankle center. In order to make two sides of the angle coincide in the surgery, namely the mechanical axis of the lower limb coincide with a connecting line from the hip center to the ankle center, the $\angle AHB$ refers to an angle rotated with the hinge point as an axis.

The $\angle AHB$ is calculated in two parts, including an included angle$_1$ between the segment AH and the x axis, and an included angle$_2$ between the segment BH and the y axis. The $\angle AHB$ is as shown by Eqs. (6), (7) and (8):

$$\angle AHB = angle_1 + angle_2 \tag{6};$$

$$angle_1 = \left( \arctan \frac{dy_1}{dx_1} \right) \cdot \frac{180}{\pi} \tag{7};$$

and

$$angle_2 = \left( \arctan \frac{dx_2}{dy_2} \right) \cdot \frac{180}{\pi} \tag{8}$$

For surgery planning at a tibial end, regardless of a closed-wedge HTO or an HTO in which the lower half part of the image is rotated, the upper half part and the lower half part of the image are segmented according to a connecting line from the hinge point to the surgery point, as shown in Eq. (9):

$$Y = k_2 X + b_2 \tag{9}.$$

The origin is rotated as shown in FIG. 11. A modeling method in a polar coordinate system is used to rotate the image. The point $v$ is rotated around the origin for an angle $\theta$ to obtain the point $v'$. Assuming that the point $v$ has a coordinate $(u, w)$, then the point $v'$ has a coordinate $(u', w')$. Assuming that a distance from the origin to the point $v$ is $r$, and an included angle between a vector from the origin to the point $v$ and the x axis is $\phi$ then $v$ and $v'$ can be expressed as Eq. (10) and Eq. (11) respectively, with a matrix form being expressed as Eq. (12):

$$\begin{cases} u = r \cos \phi \\ w = r \sin \phi \end{cases} \tag{10};$$

$$\begin{cases} u' = r\cos(\theta+\phi) \\ w' = r\sin(\theta+\phi) \end{cases} \quad (11);$$

and

$$\begin{bmatrix} u' \\ w' \end{bmatrix} = \begin{bmatrix} \cos\theta & \sin\theta \\ -\sin\theta & \cos\theta \end{bmatrix}\begin{bmatrix} u \\ w \end{bmatrix} \quad (12).$$

Assuming that the hinge point has a coordinate $(x_0, y_0)$, then when the image is rotated around the hinge point, the hinge point first moves to the origin, as shown in Eq. (13), where x,y is a distance from the hinge point $(x_0,y_0)$ to the origin, and after the hinge point $(x_0,y_0)$ moves to the origin, $(x'_0, y'_0)$ is obtained:

$$\begin{cases} x'_0 = x + tx_0 \\ y'_0 = y + ty_0 \end{cases} \quad (13).$$

Eq. (14) shows homogeneous coordinates after a translation matrix and a rotation matrix of the image are introduced. The matrix becomes a form 3×3:

$$\begin{bmatrix} x'_0 \\ y'_0 \\ 1 \end{bmatrix} = \begin{bmatrix} 1 & 0 & tx_0 \\ 0 & 1 & ty_0 \\ 0 & 0 & 1 \end{bmatrix}\begin{bmatrix} x_0 \\ y_0 \\ 1 \end{bmatrix} \quad (14).$$

where, $t$ is an offset coefficient.
After returning to the origin, the hinge point is rotated by $\theta$ degrees with the original as a center, as shown in Eq. (15):

$$\begin{bmatrix} x'_0 \\ y'_0 \\ 1 \end{bmatrix} = \begin{bmatrix} \cos\theta & \sin\theta & 0 \\ -\sin\theta & \cos\theta & 0 \\ 0 & 0 & 1 \end{bmatrix}\begin{bmatrix} x_0 \\ y_0 \\ 1 \end{bmatrix} \quad (15).$$

In this case, the hinge point is translated to an original position, as shown in Eq. (16):

$$\begin{bmatrix} x'_0 \\ y'_0 \\ 1 \end{bmatrix} = \begin{bmatrix} 1 & 0 & -tx \\ 0 & 1 & -ty \\ 0 & 0 & 1 \end{bmatrix}\begin{bmatrix} x_0 \\ y_0 \\ 1 \end{bmatrix} \quad (16).$$

At last, a rotation matrix of the lower half part of the image around the hinge point can be obtained, as shown in Eq. (17):

$$M = \begin{bmatrix} \cos\theta & \sin\theta & (1-\cos\theta)tx - ty\sin\theta \\ -\sin\theta & \cos\theta & tx\sin\theta + ty(1-\cos\theta) \\ 0 & 0 & 1 \end{bmatrix} \quad (17).$$

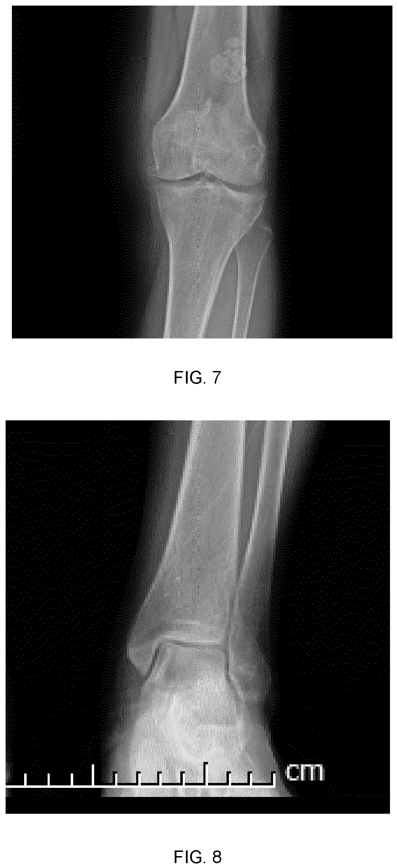

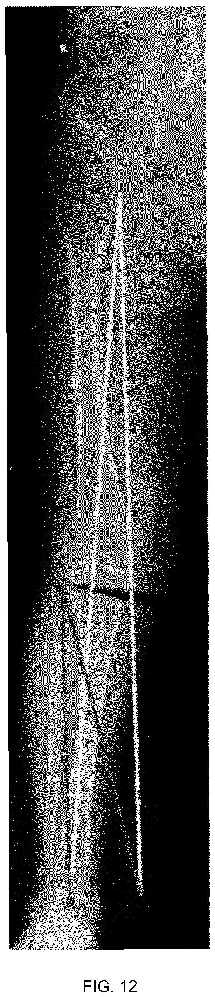

According to the corrective angle between the line 2 and the line 3 in FIG. 8, the lower half part of the image is rotated with the hinge point as a center of rotation to obtain a surgery planning image, as shown in FIG. 12.

Regarding the DFO, the present disclosure takes a lateral opening DFO as an example. A coordinate of the hip center, a coordinate of the ankle center, a coordinate of the hinge point, a coordinate of the surgery point and a coordinate of the Fujisawa point are obtained first. A connecting line from the ankle center to the Fujisawa point and an extended line form a target mechanical axis. As shown in FIG. 12, the target mechanical axis is called a line 1', and the hinge point and the hip center form a line 2'. With the hinge point as a center, and a length of the line 2' as a radius, rotation is made to the line 1' to obtain a line 3'. In this case, the hip center coincides with the line 1'. An included angle between the line 3' and the line 2' is an angle to be corrected. The included angle is solved by Eq. (6), Eq. (7) and Eq. (8).

As shown in Eq. (18), the line 1' (target mechanical axis) determined by the ankle center and the Fujisawa point has a linear equation:

$$y_5 = k_5 x_5 + b_5 \quad (18).$$

For surgery planning on a femoral end, an upper half part of the image is rotated. The upper half part and the lower half part of the image are segmented according to a connecting line from the hinge point to the surgery point, as shown in FIG. (19):

$$y_6 = k_6 x_6 + b_6 \quad (19).$$

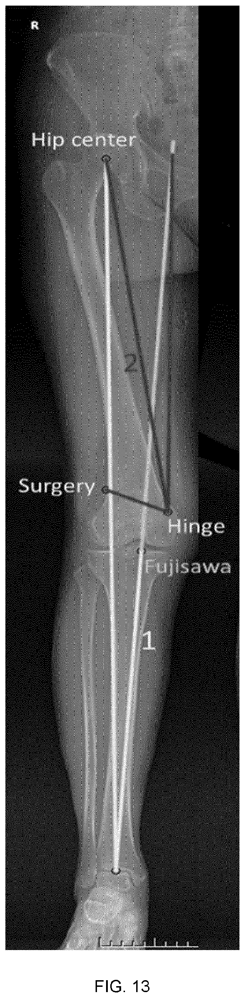

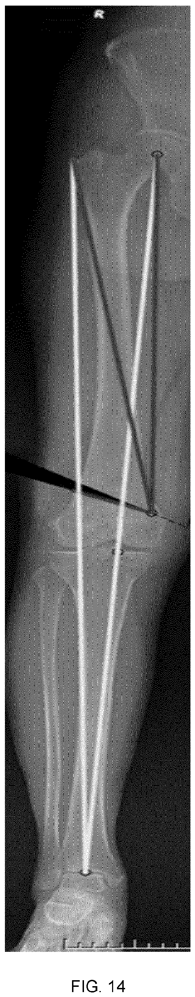

The method for solving the line 2' and the line 3' and the method for rotating the image are the same as those in the medial opening HTO. According to the corrective angle between the line 2' and the line 3' in FIG. 13, the upper half part of the image is rotated with the hinge point as a center of rotation to obtain a preoperative planning image in the lateral opening DFO, as shown in FIG. 14.

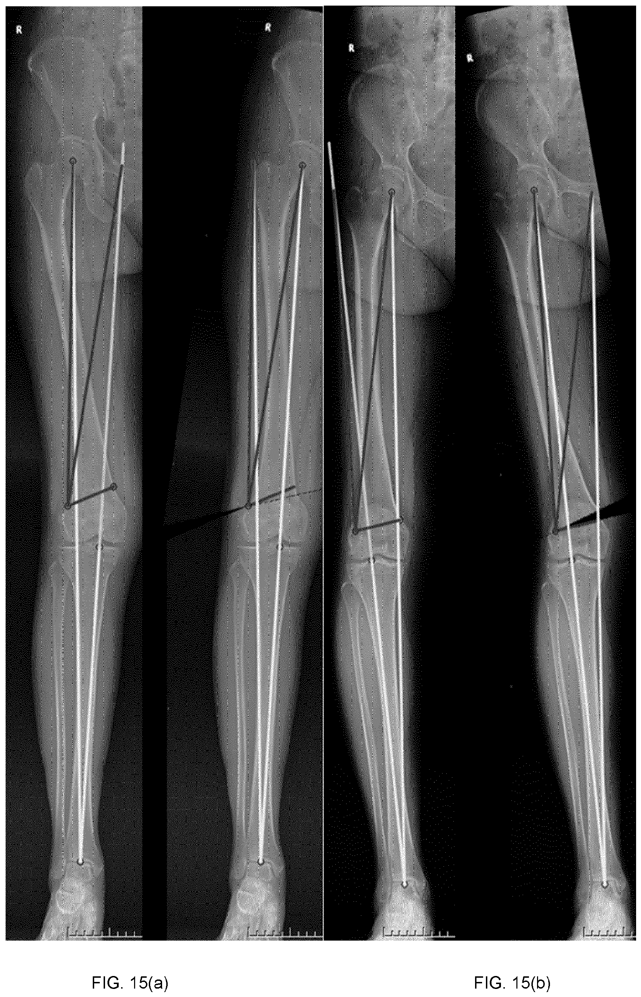

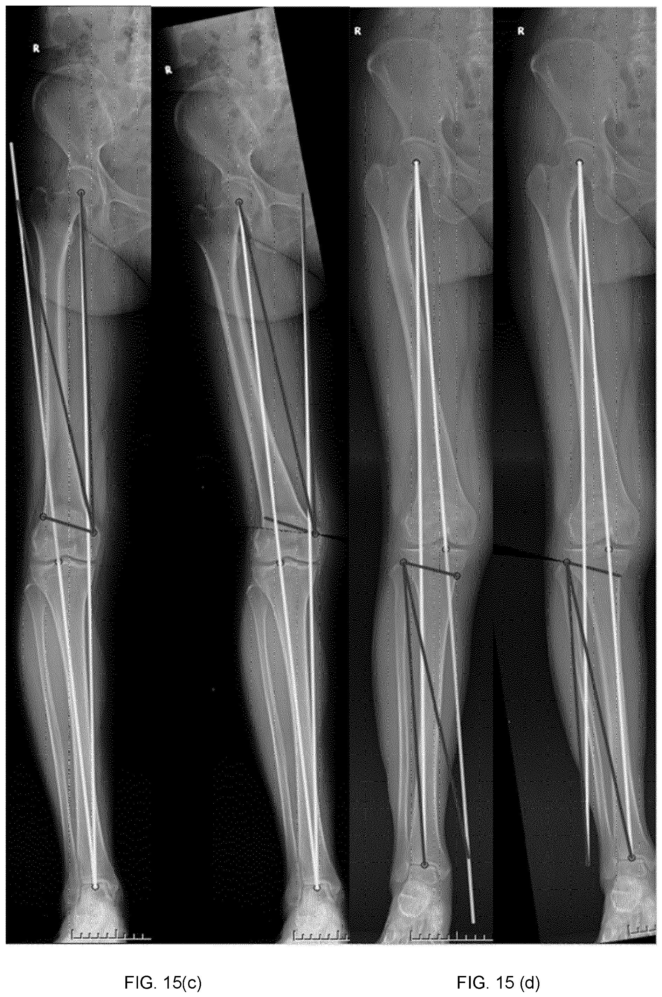

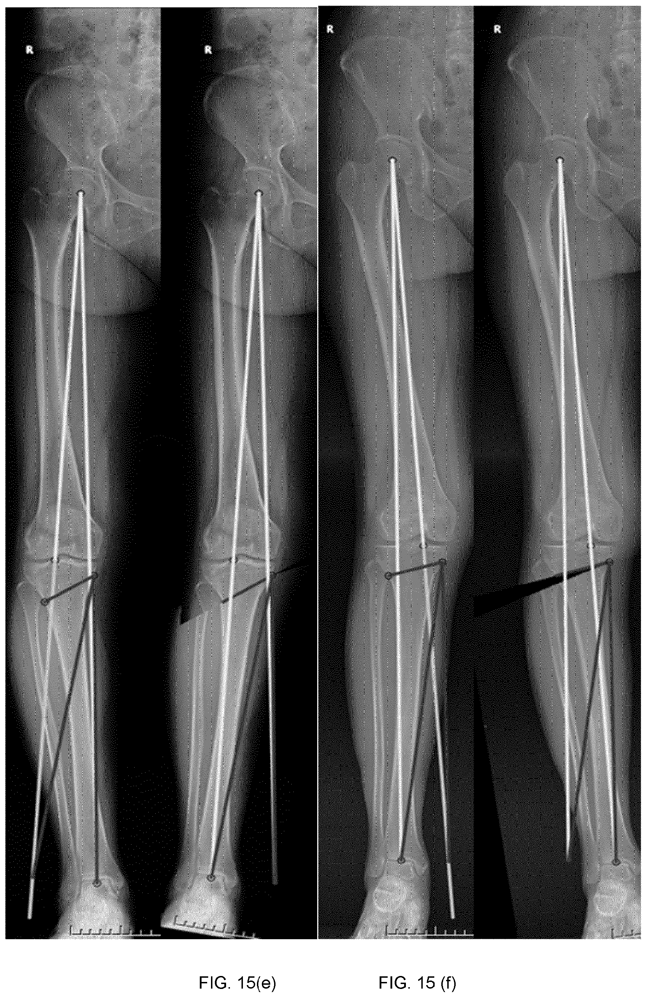

With coordinate calculation and image processing, the present disclosure realizes preoperative planning for various surgeries on the DFO and the HTO. FIG. 15 shows preoperative planning and effect sketches of the (a) medial closing DFO, (b) medial opening DFO, (c) lateral closing DFO, (d) medial closing HTO, (e) lateral closing HTO and (f) lateral opening HTO.

The present disclosure reduces a key point detection range by segmenting key regions. With image processing, the present disclosure converts size information of the ruler into the pixel pitch, thus providing a reference to determine a size in the surgery. The present disclosure selects the key regions and detects the key points with the YOLOX and the PFDNet. With the coordinate calculation and the image processing, the present disclosure automatically implements bone cutting and bone rotation in the simulated surgery, and displays a final effect.

The present disclosure further provides an automatic planning system for a simulated knee surgery based on deep learning, including: an image preprocessing unit, a key point extraction unit, and an automatic planning unit.

The image preprocessing unit is configured to acquire a medical image, and convert the pixel pitch in the medical image into an actual length.

The key point extraction unit is configured to select a key regional image in the medical image, and perform object detection on the key regional image to extract key points.

The automatic planning unit is configured to calculate a corrective angle with coordinates of the extracted key points by labeling the key points and a mechanical axis, calculate a rotated angle, simulate bone cutting and bone rotation to obtain a surgery planning image, and perform automatic planning on an HTO and a DFO.

**Claims**

1. An automatic planning method for a simulated knee surgery based on deep learning, comprising:

   acquiring a medical image of a knee joint, and converting a pixel pitch in the medical image into an actual length;
   selecting a key regional image in the medical image, and performing object detection on the key regional image to extract key points based on deep learning; and
   calculating a corrective angle with coordinates of the extracted key points by labeling the key points and a mechanical axis, calculating a rotated angle, simulating bone cutting and bone rotation to obtain a surgery planning image, and performing automatic planning on a high tibial osteotomy (HTO) and a distal femoral osteotomy (DFO),
   **characterized in that,**
   the converting the pixel pitch in the medical image into the actual length comprises:

acquiring a ruler image from the medical image by, screening out white pixels in the medical image, and detecting the ruler image with a line segment detector (LSD); and

acquiring a head endpoint coordinate and a tail endpoint coordinate of a ruler in the ruler image, and performing conversion on a pixel difference value between the head endpoint coordinate and the tail endpoint coordinate ($X_A$ and $X_B$) of the ruler in an X direction and a millimeter unit to obtain a coefficient of relationship $\alpha$

$$\alpha = \frac{(X_A - X_B)/18}{10}$$

between the millimeter unit and the pixel difference value, .

2. The automatic planning method for the simulated knee surgery based on the deep learning according to claim 1, wherein the selecting a key regional image in the medical image of a knee joint, and performing object detection on the key regional image to extract key points comprises:

selecting an image comprising a hip region, a knee region and an ankle region as the key regional image;

performing, with a you only look once X (YOLOX), the object detection on the image comprising the hip region, the knee region and the ankle region;

extracting a hip center, a hinge point in the knee region, a Fujisawa point, a surgery point and an ankle center as the key points with a pulse feature disentanglement network (PFDNet).

3. The automatic planning method according to claim 2, wherein the calculating a corrective angle with coordinates of the extracted key points by labeling the key points and a mechanical axis comprises:

assuming that a connecting line from the hip center to the Fujisawa point and an extended line thereof form a target mechanical axis called a line 1, and the hinge point and the ankle center form a line 2, then making rotation to the line 1 with the hinge point as a center and a length of the line 2 as a radius to obtain a line 3, wherein the ankle center coincides with the line 1, and an included angle between the line 3 and the line 2 is an angle to be corrected in the HTO; and

assuming that a connecting line from the ankle center to the Fujisawa point and an extended line thereof form a target mechanical axis called a line 1', and the hinge point and the hip center form a line 2', then making rotation to the line 1' with the hinge point as a center and a length of the line 2' as a radius to obtain a line 3', wherein the hip center coincides with the line 1', and an included angle between the line 3' and the line 2' is an angle to be corrected in the DFO.

4. An automatic planning system for a simulated knee surgery based on deep learning, comprising:

an image preprocessing unit configured to acquire a medical image of a knee joint, and to convert a pixel pitch in the medical image into an actual length;

a key point extraction unit configured to select a key regional image in the medical image, and perform object detection on the key regional image to extract key points; and

an automatic planning unit configured to calculate a corrective angle with coordinates of the extracted key points by labeling the key points and a mechanical axis, calculate a rotated angle, simulate bone cutting and bone rotation to obtain a surgery planning image, and perform automatic planning on a high tibial osteotomy (HTO) and a distal femoral osteotomy (DFO),

**characterized in that,**

the system is further configured to, when converting the pixel pitch in the medical image into the actual length, to acquire a ruler image from the medical image by screening out white pixels in the medical image, and to detect the ruler image with a line segment detector (LSD); and

to acquire a head endpoint coordinate and a tail endpoint coordinate of a ruler in the ruler image, and perform conversion on a pixel difference value between the head endpoint coordinate and the tail endpoint coordinate (XA and XB) of the ruler in an X direction and a millimeter unit to obtain a coefficient of relationship $\alpha$ between the

$$\alpha = \frac{(X_A - X_B)/18}{10}$$

millimeter unit and the pixel difference value, .

**Patentansprüche**

1. Ein automatisches Planungsverfahren für eine simulierte Knieoperation basierend auf Deep Learning, umfassend:

   Erfassen eines medizinischen Bildes eines Kniegelenks und Umwandeln eines Pixelabstands im medizinischen Bild in eine tatsächliche Länge;
   Auswählen eines Schlüsselbereichsbildes im medizinischen Bild und Durchführen einer Objekterkennung auf dem Schlüsselbereichsbild, um Schlüsselpunkte auf der Grundlage von Deep Learning zu extrahieren; und
   Berechnen eines Korrekturwinkels mit Koordinaten der extrahierten Schlüsselpunkte durch Markieren der Schlüsselpunkte und einer mechanischen Achse, Berechnen eines Rotationswinkels, Simulieren des Knochenschneidens und der Knochenrotation, um ein Operationsplanungsbild zu erhalten, sowie automatisches Planen einer hohen Tibiakorrekturosteotomie (HTO) und einer distalen Femurosteotomie (DFO),
   **dadurch gekennzeichnet, dass**
   das Umwandeln des Pixelabstands im medizinischen Bild in die tatsächliche Länge Folgendes umfasst:

   Erfassen eines Linealbildes aus dem medizinischen Bild durch Herausfiltern von weißen Pixeln im medizinischen Bild und Erkennen des Linealbildes mit einem Liniensegmentdetektor (LSD); und
   Ermitteln einer Kopfendpunkt-Koordinate und einer Schwanzendpunkt-Koordinate eines Lineals im Linealbild sowie Durchführen einer Umwandlung eines Pixeldifferenzwerts zwischen der Kopfendpunkt-Koordinate und der Schwanzendpunkt-Koordinate ($X_A$ und $X_B$) des Lineals in X-Richtung und einer Millimetereinheit, um einen Umrechnungsfaktor $\alpha$ zwischen der Millimetereinheit und dem Pixeldifferenzwert,

   $$\alpha = \frac{(X_A - X_B)/18}{10}$$ , zu erhalten.

2. Das automatische Planungsverfahren für die simulierte Knieoperation basierend auf Deep Learning nach Anspruch 1, wobei das Auswählen eines Schlüsselbereichsbildes im medizinischen Bild eines Kniegelenks und das Durchführen einer Objekterkennung auf dem Schlüsselbereichsbild zur Extraktion von Schlüsselpunkten Folgendes umfasst:

   Auswählen eines Bildes, das einen Hüftbereich, einen Kniebereich und einen Knöchelbereich umfasst, als Schlüsselbereichsbild;
   Durchführen der Objekterkennung auf dem Bild, das den Hüftbereich, den Kniebereich und den Knöchelbereich umfasst, mit einem "You Only Look Once X" (YOLOX);
   Extrahieren eines Hüftzentrums, eines Scharnierpunkts im Kniebereich, eines Fujisawa-Punkts, eines Operationspunkts und eines Knöchelzentrums als Schlüsselpunkte mit einem Pulse Feature Disentanglement Network (PFDNet).

3. Das automatische Planungsverfahren nach Anspruch 2, wobei das Berechnen eines Korrekturwinkels mit Koordinaten der extrahierten Schlüsselpunkte durch Markieren der Schlüsselpunkte und einer mechanischen Achse Folgendes umfasst:

   Annehmen, dass eine Verbindungslinie vom Hüftzentrum zum Fujisawa-Punkt und eine Verlängerungslinie davon eine Zielmechanikachse bilden, die als Linie 1 bezeichnet wird, und dass der Scharnierpunkt und das Knöchelzentrum eine Linie 2 bilden, dann Durchführen einer Rotation der Linie 1 mit dem Scharnierpunkt als Zentrum und der Länge der Linie 2 als Radius, um eine Linie 3 zu erhalten, wobei das Knöchelzentrum mit der Linie 1 zusammenfällt, und dass ein eingeschlossener Winkel zwischen der Linie 3 und der Linie 2 ein zu korrigierender Winkel in der HTO ist; und
   Annehmen, dass eine Verbindungslinie vom Knöchelzentrum zum Fujisawa-Punkt und eine Verlängerungslinie davon eine Zielmechanikachse bilden, die als Linie 1' bezeichnet wird, und dass der Scharnierpunkt und das Hüftzentrum eine Linie 2' bilden, dann Durchführen einer Rotation der Linie 1' mit dem Scharnierpunkt als Zentrum und der Länge der Linie 2' als Radius, um eine Linie 3' zu erhalten, wobei das Hüftzentrum mit der Linie 1' zusammenfällt, und dass ein eingeschlossener Winkel zwischen der Linie 3' und der Linie 2' ein zu korrigierender Winkel in der DFO ist.

4. Ein automatisches Planungssystem für eine simulierte Knieoperation basierend auf Deep Learning, umfassend:

eine Bildvorverarbeitungseinheit, die dazu konfiguriert ist, ein medizinisches Bild eines Kniegelenks zu erfassen und einen Pixelabstand im medizinischen Bild in eine tatsächliche Länge umzuwandeln;

eine Schlüsselpunkt-Extraktionseinheit, die dazu konfiguriert ist, ein Schlüsselbereichsbild im medizinischen Bild auszuwählen und eine Objekterkennung auf dem Schlüsselbereichsbild durchzuführen, um Schlüsselpunkte zu extrahieren; und

eine automatische Planungseinheit, die dazu konfiguriert ist, einen Korrekturwinkel mit Koordinaten der extrahierten Schlüsselpunkte durch Markieren der Schlüsselpunkte und einer mechanischen Achse zu berechnen, einen Rotationswinkel zu berechnen, das Knochenschneiden und die Knochenrotation zu simulieren, um ein Operationsplanungsbild zu erhalten, sowie eine automatische Planung einer hohen Tibiakorrekturosteotomie (HTO) und einer distalen Femurosteotomie (DFO) durchzuführen,

**dadurch gekennzeichnet, dass**

das System ferner dazu konfiguriert ist, beim Umwandeln des Pixelabstands im medizinischen Bild in die tatsächliche Länge, ein Linealbild aus dem medizinischen Bild zu erfassen, indem weiße Pixel im medizinischen Bild herausgefiltert werden, und das Linealbild mit einem Liniensegmentdetektor (LSD) zu erkennen; und eine Kopfendpunkt-Koordinate und eine Schwanzendpunkt-Koordinate eines Lineals im Linealbild zu ermitteln und eine Umwandlung eines Pixeldifferenzwerts zwischen der Kopfendpunkt-Koordinate und der Schwanzendpunkt-Koordinate (XA und XB) des Lineals in X-Richtung und einer Millimetereinheit durchzuführen, um einen

Umrechnungsfaktor $\alpha$ zwischen der Millimetereinheit und dem Pixeldifferenzwert, $\alpha = \dfrac{(X_A - X_B)/18}{10}$ , zu erhalten.

## Revendications

1. Un procédé de planification automatique d'une chirurgie simulée du genou basé sur l'apprentissage profond, comprenant :

   l'acquisition d'une image médicale d'une articulation du genou et la conversion d'un pas de pixel dans l'image médicale en une longueur réelle ;

   la sélection d'une image de région clé dans l'image médicale et la réalisation d'une détection d'objets sur l'image de région clé afin d'extraire des points clés sur la base de l'apprentissage profond ; et

   le calcul d'un angle de correction avec les coordonnées des points clés extraits en marquant les points clés et un axe mécanique, le calcul d'un angle de rotation, la simulation de la coupe osseuse et de la rotation osseuse pour obtenir une image de planification chirurgicale, et la planification automatique d'une ostéotomie tibiale haute (HTO) et d'une ostéotomie fémorale distale (DFO),

   **caractérisé en ce que**

   la conversion du pas de pixel dans l'image médicale en longueur réelle comprend :

   l'acquisition d'une image de règle à partir de l'image médicale en filtrant les pixels blancs dans l'image médicale et la détection de l'image de règle à l'aide d'un détecteur de segments de ligne (LSD) ; et

   l'acquisition d'une coordonnée de l'extrémité de tête et d'une coordonnée de l'extrémité de queue d'une règle dans l'image de règle, et la conversion d'une valeur de différence de pixels entre la coordonnée de l'extrémité de tête et la coordonnée de l'extrémité de queue ($X_A$ et $X_B$) de la règle dans une direction X et une unité en millimètres afin d'obtenir un coefficient de relation $\alpha$ entre l'unité en millimètres et la valeur de différence de pixels, $\alpha = \dfrac{(X_A - X_B)/18}{10}$ .

2. Le procédé de planification automatique d'une chirurgie simulée du genou basé sur l'apprentissage profond selon la revendication 1, dans lequel la sélection d'une image de région clé dans l'image médicale d'une articulation du genou et la réalisation d'une détection d'objets sur l'image de région clé pour extraire des points clés comprend :

   la sélection d'une image comprenant une région de la hanche, une région du genou et une région de la cheville en tant qu'image de région clé ;

   la réalisation de la détection d'objets sur l'image comprenant la région de la hanche, la région du genou et la région de la cheville à l'aide de "You Only Look Once X" (YOLOX) ;

   l'extraction d'un centre de hanche, d'un point de charnière dans la région du genou, d'un point de Fujisawa, d'un point d'intervention et d'un centre de cheville en tant que points clés avec un réseau de désentrelacement des caractéristiques d'impulsion (PFDNet).

3. Le procédé de planification automatique selon la revendication 2, dans lequel le calcul d'un angle de correction avec les coordonnées des points clés extraits en marquant les points clés et un axe mécanique comprend :

le fait de supposer qu'une ligne de connexion entre le centre de la hanche et le point de Fujisawa et une ligne étendue de celle-ci forment un axe mécanique cible appelé ligne 1, et que le point de charnière et le centre de la cheville forment une ligne 2, puis effectuer une rotation de la ligne 1 avec le point de charnière comme centre et la longueur de la ligne 2 comme rayon pour obtenir une ligne 3, dans laquelle le centre de la cheville coïncide avec la ligne 1, et un angle inclus entre la ligne 3 et la ligne 2 est un angle à corriger dans la HTO ; et

le fait de supposer qu'une ligne de connexion entre le centre de la cheville et le point de Fujisawa et une ligne étendue de celle-ci forment un axe mécanique cible appelé ligne 1', et que le point de charnière et le centre de la hanche forment une ligne 2', puis effectuer une rotation de la ligne 1' avec le point de charnière comme centre et la longueur de la ligne 2' comme rayon pour obtenir une ligne 3', dans laquelle le centre de la hanche coïncide avec la ligne 1', et un angle inclus entre la ligne 3' et la ligne 2' est un angle à corriger dans la DFO.

4. Un système de planification automatique pour une chirurgie simulée du genou basé sur l'apprentissage profond, comprenant :

une unité de prétraitement d'image, configurée pour acquérir une image médicale d'une articulation du genou et convertir un pas de pixel dans l'image médicale en une longueur réelle ;

une unité d'extraction de points clés, configurée pour sélectionner une image de région clé dans l'image médicale et effectuer une détection d'objets sur l'image de région clé afin d'extraire des points clés ; et

une unité de planification automatique, configurée pour calculer un angle de correction avec les coordonnées des points clés extraits en marquant les points clés et un axe mécanique, calculer un angle de rotation, simuler la coupe osseuse et la rotation osseuse pour obtenir une image de planification chirurgicale, et effectuer une planification automatique d'une ostéotomie tibiale haute (HTO) et d'une ostéotomie fémorale distale (DFO), **caractérisé en ce que**

le système est en outre configuré pour, lors de la conversion du pas de pixel dans l'image médicale en une longueur réelle, acquérir une image de règle à partir de l'image médicale en filtrant les pixels blancs dans l'image médicale, et détecter l'image de règle à l'aide d'un détecteur de segments de ligne (LSD) ; et

acquérir une coordonnée de l'extrémité de tête et une coordonnée de l'extrémité de queue d'une règle dans l'image de règle, et effectuer une conversion d'une valeur de différence de pixels entre la coordonnée de l'extrémité de tête et la coordonnée de l'extrémité de queue (XA et XB) de la règle dans une direction X et une unité en millimètres afin d'obtenir un coefficient de relation $\alpha$ entre l'unité en millimètres et la valeur de différence de

$$\alpha = \frac{(X_A - X_B)/18}{10}$$

pixels, .

FIG. 1

FIG. 2

FIG. 3

FIG.4

FIG.5

FIG. 6

15

FIG. 7

FIG. 8

FIG. 9

FIG. 10

FIG. 11

FIG. 12

FIG. 13

FIG. 14

FIG. 15(a)

FIG. 15(b)

FIG. 15(c)                    FIG. 15 (d)

FIG. 15(e)          FIG. 15 (f)

**EP 4 427 696 B1**

REFERENCES CITED IN THE DESCRIPTION

**Patent documents cited in the description**

- WO 2022007342 A1